# EUROPÄISCHE PATENTSCHRIFT

(11) **EP 0 995 097 B1**
(45) Veröffentlichungstag und Bekanntmachung des Hinweises auf die Patenterteilung: **21.05.2003**
(21) Anmeldenummer: 98925437.0
(22) Anmeldetag: 24.03.1998
(51) Int. Cl.: G01N 1/28, C12M 3/08

(54) **VORRICHTUNG SOWIE VERFAHREN ZUR ISOLIERUNG VON ZELLMATERIAL AUS EINEM GEWEBEVERBAND UND/ODER EINER FLÜSSIGKEIT**
DEVICE AND METHOD FOR ISOLATING CELL MATERIAL OUT OF A TISSUE MEDIUM AND/OR A LIQUID
DISPOSITIF ET PROCEDE D'ISOLATION DE MATIERE CELLULAIRE DANS UNE ENVELOPPE TISSULAIRE ET/OU UN LIQUIDE

(30) Priorität: 08.07.1997 DE 19729028
(43) Veröffentlichungstag der Anmeldung: 26.04.2000
(73) Patentinhaber: FRAUNHOFER-GESELLSCHAFT ZUR FÖRDERUNG DER ANGEWANDTEN FORSCHUNG E.V., 80636 München (DE)
(72) Erfinder: DOBLER, Hannes, D-71227 Rutesheim (DE); KUHN, Claus, D-73105 Dürnau (DE); LINDNER, Hans, D-70567 Stuttgart (DE); KIESEWETTER, Stefan, D-73760 Ostfildern (DE); BERNHAGEN, Jürgen, D-72074 Tübingen (DE); TOLLE, Gabriele, D-69190 Walldorf/Baden (DE); TOVAR, Günter, D-70563 Stuttgart (DE)
(86) Internationale Anmeldenummer: DE9800864
(87) Internationale Veröffentlichungsnummer: WO99002958

(56) Entgegenhaltungen:
- EP-A- 0 590 504
- GB-A- 2 100 137
- US-A- 3 941 317
- US-A- 4 350 768

## Beschreibung

### Technisches Gebiet

Die Erfindung bezieht sich auf eine Vorrichtung sowie ein Verfahren zur Isolierung von Zellmaterial aus einem Gewebeverband und/oder einer Flüssigkeit mit einem oben offenen Behältnis, in das das zu isolierende Zellmaterial im Gewebeverband und/oder in der Flüssigkeit einbringbar ist.

### Stand der Technik

Zur medizinischen Diagnose von Krankheitsbildern sowie auch zu wissenschaftlichen Untersuchungszwecken, insbesondere im biomedizinischen Bereich, werden einzelne Zellen oder Zellverbände analysiert. Voraussetzung für die an Zellen durchzuführenden Analyseverfahren ist die Isolation einzelner Zellen aus Gewebeverbänden bzw. aus Flüssigkeiten, in denen die einzelnen Zellen in ihrer natürlichen Form vorkommen. In vielen Fällen werden Zellen für die Zelluntersuchung aus biologischem Weichgewebe, wie beispielsweise der Milz, Leber sowie auch aus härterem Gewebe, der Haut oder Haaren entnommen. Häufig werden auch bei der Blut- oder Urinuntersuchung Zellen aus Flüssigkeiten isoliert, die anschließend beispielsweise auf Krankheitserreger hin untersucht werden. Derartige Untersuchungen erstrecken sich auch auf in Körpersekreten, Abstrichen oder sonstigen körpereigenen Flüssigkeiten enthaltenen Zellen, die jedoch, wie in allen vorstehend genannten Fällen, aus den einzelnen Gewebeverbänden bzw. Flüssigkeiten zu isolieren sind.

Gegenwärtig sind jedoch keine standardisierten Verfahren zur Zellisolierung bekannt. Bislang bedient man sich vorwiegend der manuellen Abtrennung einzelner Zellen oder Zellkonglumeraten aus Gewebeverbänden und/oder Flüssigkeiten, so daß zur Extraktion von Zellen aus Gewebe, beispielsweise aus der Milz, verschiedene, individuell entwickelte und dem Geschick der jeweils durchführenden Person angepaßte manuelle Methoden angewendet werden. Auf diese Weise haben sich im Laufe der Zeit unterschiedliche Zellisolierungstechniken ausgebildet, die sich von Labor zu Labor unterschiedlich entwickelt haben. Auch sind aus der einschlägigen Literatur keine hilfreichen Hinweise zu entnehmen, aus denen allgemein übliche oder gar genormte Verfahren zur universellen Zellisolierung abgeleitet werden können.

Zur Herstellung einer Einzelzellsuspension für die Untersuchung eines bestimmten Zelltypes sind eine Reihe von hintereinander durchzuführenden Arbeitsschritten erforderlich, die bis heute ausschließlich manuell erfolgen.

Zur Analyse von Zellen, die in Gewebeverbänden vorkommen, muß zunächst ein Gewebestück, beispielsweise aus Milz oder Leber, in einer gewünschten Größe abgetrennt werden. Die entnommene Gewebeprobe wird für gewöhnlich in ein Medium gegeben, das zusammen mit der Probe in eine Petrischale eingebracht wird. Nachfolgend wird die in dem Medium befindliche Gewebeprobe mit Hilfe geeigneter Werkzeuge mechanisch zerdrückt und zerkleinert, wodurch die zu analysierenden Zellen besser aus dem Gewebe herausgelöst werden können. Die sich in dem Medium anreichernden, aus den einzelnen zerkleinerten Gewebestückchen herausgelösten Zellen bzw. Zellkonglumerate, sind nun als Suspension aus dem in der Petrischale befindlichen Medium zu entnehmen. Dieser Trennvorgang erfolgt für gewöhnlich unter Verwendung einer Pipette, deren Öffnung jedoch leicht durch abgetrennte kleinere Gewebestückchen verstopft werden kann. Das Abpipettieren der Zellsuspension erfordert von der jeweiligen Person daher ein besonderes handwerkliches Geschick, da die isolierten Zellen zusammen mit den durch den mechanischen Herauslösevorgang zerkleinerten Gewebestückchen in dem Medium vorliegen.

Die durch Abpipettieren gewonnene Zellsuspension wird abermals in ein Gefäß verbracht, in dem die zusammen mit den Zellen entnommenen kleineren Gewebestückchen aufgrund Ihrer größeren Masse am Gefäßboden durch Sedimentationsprozesse absitzen können. Abermals wird der vorstehend beschriebene Pipettiervorgang wiederholt, um auf diese Weise eine an Zellen angereicherte Suspension zu erhalten. Je nach Art, Größe und Anteil von in der Suspension vorliegenden, abgetrennten kleinen Gewebestückchen muß der auf Sedimentation beruhende Trennvorgang öfters wiederholt werden. Dieser Vorgang kann auch durch den Einsatz von Zentrifugen unterstützt werden.

Zum Auflösen und Trennen von in der Suspension vorliegenden Zellverbänden in einzelne Zellen wird die an Zellmaterial angereicherte Suspension mehrmals ein- und abpipettiert, so daß möglichst viele Zellen im isolierten Zustand in der Suspension vorliegen. Die auf diese Weise gewonnene Suspension von einzelnen, isolierten Zellen aus einem Gewebeverband werden für gewöhnlich in ein weiteres Gefäß verbracht, in das beispielsweise zum Entfernen der in der Suspension ebenfalls vorliegenden roten Blutkörperchen Ammoniumchlorid zugegeben wird.

Die vorstehend beschriebene Vorgehensweise zur Zellisolation beispielsweise von Zellen aus einem Gewebestückchen beispielsweise der Leber, hat gezeigt, daß eine Vielzahl von manuell hintereinander durchzuführenden Trennschritten durchzuführen sind, bis einzelne, isolierte Zellen erhalten werden können. Mit jedem der einzelnen Trennschritte fällt jedoch schwer zu entsorgender Restmüll an, beispielsweise die Suspensionsflüssigkeiten, die für den Trennvorgang benötigten Werkzeuge wie Pipettierspitze bzw. Trennvorrichtungen sowie auch die verwendeten Gefäße.

Zur Reduzierung des mit dem Trennvorgang verbundenen Aufwandes werden in vielen Labors, beispielsweise in diagnostischen PCR-Labors, auf die spezifische Herstellung von Einzelzellsuspensionen verzichtet und statt dessen der Gesamtgewebeverband aufgeschlossen. Dies mag zwar eine Zeitersparnis mit sich führen, ist jedoch mit dem Nachteil verbunden, daß uneinheitliche Homogenisate mit einer Vielzahl von Zelltypen, Gewebebruchstücken und häufig Hemmstoffen für die nachfolgende Analytik entstehen.

Aus der EP 0 590 504 A1 geht eine gattungsgemäße Trennvorrichtung hervor, die aus einem Glasbehältnis 41 besteht, in das ein hohlzylinderartig ausgebildetes Element 10 einfügbar ist, das an seinem unteren Ende über eine Trennvorrichtung 20 verfügt, die Durchlaßöffnungen 28, 38 aufweist, durch die Zellsuspension hindurchtritt, während das hohlzylinderförmige Element 10 in das mit Zellsuspension 45 gefüllte Gefäß 41 eingetaucht wird (siehe Figur 12).

### Darstellung der Erfindung

Der Erfindung liegt daher die Aufgabe zugrunde, eine Vorrichtung sowie ein Verfahren zur Isolierung von Zellmaterial aus einem Gewebeverband und/oder einer Flüssigkeit derart anzugeben, daß die Anzahl der durchzuführenden manuellen Prozeßschritte reduziert und die Durchführung der Zellisolierung weitgehend standardisiert wird, so daß eine zuverlässige Reproduzierbarkeit der mit der Zellisolation verbundenen Analyseergebnisse erhalten werden kann. Die mit jedem einzelnen Trennvorgang verbundenen Abfälle, wie beispielsweise Petrischale sowie allgemeine Gefäße und Werkzeuge, sollen überdies reduziert werden. Insbesondere soll die mit dem herkömmlichen Verfahren verbundene Kontamination des Arbeitsplatzes weitgehend vermieden werden.

Die Lösung der der Erfindung zugrundeliegenden Aufgabe ist im Anspruch 1 angegeben, in der eine erfindungsgemäße Vorrichtung beschrieben ist. Im Anspruch 15 ist ein erfindungsgemäßes Verfahren zur Isolierung von Zellmaterial aus einem Gewebeverband und/oder einer Flüssigkeit angegeben. Den Erfindungsgedanken vorteilhaft ausbildende Merkmale sind Gegenstand der Unteransprüche.

Der Erfindung liegt die Idee zugrunde, ein Probenträgersystem zu schaffen, das sämtliche Funktionen zur Zellisolation aus einem Gewebe sowie zur Anreicherung von Zellen aus einer Flüssigkeit integriert.

Erfindungsgemäß weist die Vorrichtung zur Isolation von Zellmaterial aus einem Gewebeverband und/oder einer Flüssigkeit mit einem oben offen ausgebildeten Behältnis, in das das zu isolierende Zellmaterial im Gewebeverband und/oder in der Flüssigkeit einbringbar ist, eine stempelartig ausgebildete Trennvorrichtung auf, die einen Betätigungsschaft vorsieht, an dem eine flächig ausgebildete Trennscheibe angebracht ist, deren Umfangsrand fluiddicht mit den Behältnisinnenwänden abschließt. Ferner weist die Trennscheibe wenigstens eine Durchlaßöffnung auf, die von einer Filtermembran überdeckt ist, und die von oben in das Behältnis einbringbar ist. Schließlich sind an der Trennscheibe Reibelemente vorgesehen, durch die bei Rotation der Trennscheibe Scherkräfte in das Material einbringbar sind.

Mit Hilfe der erfindungsgemäßen Vorrichtung ist es möglich, daß nach Probenentnahme, beispielsweise bei einem Arzt, die entnommene Probe sowie gegebenenfalls, den Prozeß unterstützende Reagenzien in das erfindungsgemäße Behältnis zum Transport einbringbar sind. Durch entsprechende Größengestaltung des Behältnisses kann zugleich die Größe der zu entnehmenden Gewebeprobe vorbestimmt werden, so daß auf diese Weise durch entsprechend am Gefäß anzubringende Markierungen dem Arzt angezeigt wird, wieviel Gewebevolumen für die jeweilige Untersuchung notwendig ist. Diese Maßnahme trägt ebenso dem Wunsch nach Standardisierung bei der Durchführung derartiger Gewebeuntersuchungen Rechnung.

Ist die Gewebeprobe in dem verschließbaren Behältnis im Analyselabor eingetroffen, so können im Labor entsprechende Reagenzien für den nachfolgenden Trennvorgang in das Behältnis eingebracht werden. Zur Zellisolation dient die stempelartig ausgebildete Trennvorrichtung, die von oben in das Gefäßinnere eingebracht wird. Die Trennvorrichtung ist erfindungsgemäß mit einer Trennscheibe ausgebildet, die bündig an der Gefäßinnenwand anliegt und über einen Betätigungsschaft senkrecht nach unten in das Gefäß absenkbar ist. Die Trennscheibe ist dabei derart ausgebildet, daß sie Durchlaßöffnungen aufweist, die jeweils mit einer Filtermembran zusätzlich überzogen sind. Die Filtermembran weist Membranöffnungen auf, die der Größe der jeweils zu isolierenden Zellen bzw. Zellverbänden entsprechen. Durch Absenken der Trennvorrichtung innerhalb des Behältnisses in Richtung der in einer Lösung vorliegenden Gewebeprobe, können die Lösung sowie die aus der Gewebeprobe herausgelösten Zellen bzw. Zellverbände durch die Filtermembran hindurch in den oberen Bereich des Behältnisses gelangen. Auf diese Weise werden in dem Behältnis zwei Raumbereiche geschaffen, die durch die Trennscheibe voneinander abgetrennt werden. Unterhalb der Trennscheibe befindet sich das in der Lösung vorliegende Gewebestück, wohingegen oberhalb der Trennscheibe die in Suspension vorliegenden isolierten Zellen bzw. Zellverbände enthalten sind.

Die vorzugsweise an der Unterseite der Trennscheibe angebrachten Reibelemente sind in Form von spitzen bzw. kantigen Fortsätzen ausgebildet, die durch Absenken und durch Rotation der Trennscheibe die Gewebeprobe regelrecht zerreißen, so daß die in der Gewebeprobe befindlichen Zellen bzw. Zellverbände möglichst vollständig als Suspension vorliegen und auf diese Weise durch die Filtermembran in den oberen Teil des Behältnisses hindurchtreten können.

Über den Betätigungsschaft, der sowohl als zentraler Haltestiel auf der Trennscheibe angebracht ist oder als Hohlzylinder ausgebildet ist, der mit dem peripheren Umfangsrand der Trennscheibe abschließt, kann die Trennscheibe kontrolliert in das Behältnis abgesenkt und in Rotation versetzt werden, so daß gezielte Druck- und Scherkräfte auf die in der Lösung befindliche Gewebeprobe ausgeübt werden können. Unterstützt durch die an der Trennscheibe befindlichen Reibelemente kann eine höchst effiziente Trennung der zu isolierenden Zellen bzw. Zellverbände von der Gewebeprobe realisiert werden.

Durch die räumliche Trennung der in Suspension vorliegenden Zellen und Zellverbände von der unter der Trennscheibe befindlichen zerkleinerten Gewebestücke kann mit Hilfe üblicher Pipetten die Zellsuspension oberhalb der Trennscheibe aus dem Behältnis entnommen werden, ohne der Gefahr zu unterliegen, daß kleine Gewebestücke die Pipettenöffnung verstopfen. Nach entsprechender Entnahme der Zellsuspension durch Pipettierung bzw. Dekantierung können die im Behältnis unterhalb der Trennscheibe befindlichen Gewebereststoffe und -reagenzien samt dem Behältnis entsorgt werden.

Die erfindungsgemäße Vorrichtung ermöglicht auf diese Weise ein Probenträgerund Zellenisolier-System, das trotz hoher Sterilitätsanforderungen sowohl für die Gewebeprobenaufnahme, den Transport in ein Untersuchungslabor, den gesamten Isolierungsprozeß sowie die abschließende Entsorgung geeignet ist. Durch die Vorgabe geeigneter Volumengrößen der Behältnisse können Gewebeanalysen besser standardisiert werden, so daß die Analysenergebnisse mit einer zuverlässigeren Reproduzierbarkeit erreicht werden können.

Durch die erfindungsgemäß ausgebildete Trennvorrichtung ist es zum einen möglich, die Prozeßgeschwindigkeit, mit der die Zellisolierung durchgeführt wird, erheblich zu steigern und zum anderen, den manuellen Aufwand zum Durchführen der Zellisolierung zu verringern. Da der gesamte Isolierungsvorgang in ein- und demselben Behältnis stattfindet, können Risiken zur Kreuzkontamination mit anderen Proben oder Personen oder Gegenständen auf ein Minimum reduziert werden.

In vorteilhafter Weise ist der für das Handhaben der Trennvorrichtung vorgesehene Betätigungsschaft sowohl manuell als auch automatisiert zu bedienen. Neben der Möglichkeit, den Betätigungsschaft lösbar fest an einer, das Behältnis abschließenden Verschlußkappe anzubringen und auf diese Weise eine vereinfachte; manuelle Handhabung der Trennvorrichtung zu realisieren, kann der Betätigungschaft zudem einen Flansch zur automatischen Betätigung mittels einer geeigneten Handhabungsvorrichtung, bspw. ein Roboterarm, vorsehen. Mit Hilfe des Einsatzes von Bewegungsautomaten ist es möglich, unter Verwendung des erfindungsgemäßen Probenträgersystems die Zellisolierung vollautomatisch durchzuführen. Dies wiederum kommt dem Ziel einer standardisierten Methode zur Zellisolierung und damit verbunden mit einer hohen Reproduzierbarkeit näher.

Auch im Hinblick des steigenden Umweltbewußtseins trägt das erfindungsgemäße Probenträgersystem dazu bei, die zu entsorgenden Abfallprodukte durch Verwendung nur eines einzigen Behältnisses zu minimieren.

### Kurze Beschreibung der Zeichnungen

Die Erfindung wird anhand eines Ausführungsbeispiels unter Bezugnahme auf die Zeichnung exemplarisch beschrieben. Es zeigen:
- Fig. 1: Darstellung des Probenträgersystems und
- Fig. 2: Sequenzdarstellungen zur Anwendung des Probenträgersystems.

### Beschreibung eines Ausführungsbeispiels und gewerbliche Anwendbarkeit

Das erfindungsgemäße Probenträgersystem besteht aus einem vorzugsweise zylinderförmig ausgebildeten Behältnis 1, das gleichsam einem Becher eine obere Öffnung aufweist. Durch die obere Öffnung des Behältnisses 1, die im gezeigten Ausführungsbeispiel mit einem Schraubdeckel 4 abgeschlossen ist, ist eine Trennvorrichtung einbringbar, die aus einer Trennscheibe 2 und einem Betätigungsschaft 3 besteht. Der Betätigungsschaft 3 sitzt zentrisch auf der Trennscheibe 2 und ist mit dieser fest verbunden. Auf der anderen Seite des Betätigungsschaftes 2 ist dieser lösbar fest mit dem Schraubdeckel 4 verbindbar, der das Behältnis 1 fluiddicht abschließt. Vorzugsweise weist der Schraubdeckel 4 an seiner oberen Fläche ein Septum auf, das zum Durchstechen von Pipettiernadeln zur Entnahme der Zellsuspension geeignet ist.

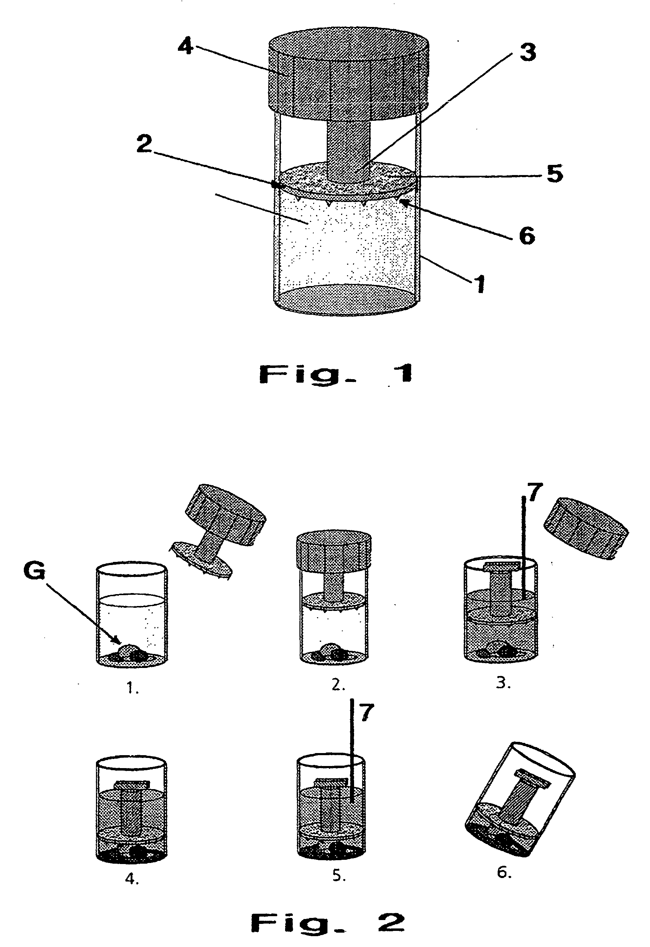

Vermittels des Betätigungsschaftes 3 können auf die Trennscheibe 2 gezielte Kraftmomente übertragen werden, indem der Betätigungsschaft 3 rotatorisch um seine Schaftachse und/oder vertikal angetrieben wird. Insbesondere ist am oberen Bereich des Betätigungsschaftes 3 ein Adapter bzw. ein Flansch für automatische Bediengeräte, wie beispielsweise Roboterarm, angebracht (nicht in der Fig. 1 dargestellt).

Die Trennscheibe 2 weist auf ihrer ringförmigen Oberfläche Durchlaßöffnungen auf, die mit einer Filtermembran 5 überdeckt sind. Die Porengröße der Filtermembran richtet sich je nach Ausgangsgewebe und Zellgröße und beträgt üblicherweise zur Untersuchung von Leberzellen ca. 100 µm.

An der Unterseite der Trennscheibe 2 sind Reibelemente 6 angebracht, die in der gezeigten Figur als spitze Kanten ausgebildet sind. Die Reibelemente 6 bohren sich durch entsprechendes Absenken der Trennvorrichtung in das unter der Trennscheibe 2 befindliche Gewebestück ein (nicht in Fig. 1 dargestellt) und zerkleinern das Gewebematerial derart, daß die im Gewebe enthaltenen Zellen und Zellkonglumerate bevorzugt aus diesen herauslösbar sind.

Das in der Fig. 1 dargestellte Behältnis ist aus leicht sterilisierbarem Kunststoff hergestellt, das zur besseren Visualisierung der im Behältnisinnem erfolgenden Trennvorgänge lichttransparent ausgebildet ist.

Alternativ zu der in der Fig. 1 ausgeführten Geometrie des Betätigungsschaftes 3 kann dieser als Hohlzylinder ausgebildet werden, der bündig am peripheren Außenumfang der Trennscheibe 2 anschließt. Durch eine derartige Ausgestaltung des Betätigungsschaftes kann die gesamte Fläche der Trennscheibe 2 für den Trennvorgang genutzt werden, d.h., auch der, in dem gezeigten Ausführungsbeispiel von dem zentrisch angebrachten Betätigungsschaft auf der Trennscheibe überdeckte Bereich wirkt als Filter.

In den Sequenzdarstellungen gemäß Fig. 2 ist eine vorteilhafte Verwendungsweise des erfindungsgemäßen Probenträgersystems dargestellt. In der ersten Sequenzdarstellung sind in das Innere des Behältnisses 1 zu analysierende Gewebestücke G eingebracht. Zusätzlich ist eine Flüssigkeit zum Lösen der in dem Gewebematerial befindlichen Zellen bzw. Zellverbände eingebracht, wie beispielsweise eine Saline-Lösung und/oder eine Ammoniumchloridlösung zum Entfernen roter Blutkörperchen.

Im zweiten Schritt wird die in das Behältnis verbrachte Gewebeprobe samt Lösung, durch eine Verschlußkappe fluiddicht verschlossen und steht für den Transport in ein Analysenlabor bereit.

Gegebenenfalls können zur Verbesserung der Trennreaktionen weitere Reagenzien in das Behältnis eingegeben werden. Hierzu ist entweder die Verschlußkappe von dem Behältnis abzunehmen und wie im gezeigten Beispiel gemäß Sequenzdarstellung 3 die weiteren Reagenzien mittels einer Pipettiervorrichtung 7 in das Innere des Behältnisses einzubringen. Alternativ ist die Verschlußkappe mit einem Septum ausgestattet, das mit Hilfe geeigneter spitz zulaufender Kanülen durchstochen werden kann, um auf diese Weise unter Vermeidung von Keimkontamination eine Flüssigkeit in das Innere des Behältnisses einzubringen.

In der Sequenzdarstellung 4 wird die erfindungsgemäße Trennvorrichtung entweder manuell oder automatisiert mit Hilfe eines Roboterarmes im Behälter nach unten gesenkt und zugleich in Rotationsbewegung versetzt, so daß die auf dem Boden des Behälters befindlichen Gewebestücke druckbeaufschlagt und unter Einwirkung von Scherkräften zerkleinert werden. Da der Außenumfang der Trennscheibe 2 fluiddicht an der Gefäßwandinnenseite anliegt, tritt die im Behältnis befindliche Flüssigkeit sowie die aus dem Gewebematerial ausgelösten Zellen bzw. Zellkonglumerate ausschließlich durch die auf der Trennscheibe aufgebrachten Filtermembran in den oberen Bereich des Behälters.

In der Sequenz 5 wird die oberhalb der Trennscheibe befindliche Zellsuspension abpipettiert. Die Gefahr des Verstopfens der Pipettenöffnung durch Gewebestückchen besteht jedoch nicht, da durch die Filtermembran keine makroskopischen Gewebestückchen gelangen können.

Schließlich fällt am Ende der Analyse gemäß Sequenz 6 lediglich das Behältnis nebst Trennvorrichtung mit dem darin befindlichen Gewebematerial als Restmüll an.

### BEZUGSZEICHENLISTE

- 1: Behältnis
- 2: Trennscheibe
- 3: Betätigungsschaft
- 4: Schraubdeckel
- 5: Filtermembran
- 6: Reibelemente
- 7: Pipettiervorrichtung
- G: Gewebestücke

## Patentansprüche

1. Vorrichtung zur Isolierung von Zellmaterial aus einem Gewebeverband und/oder einer Flüssigkeit mit einem oben offenen Behältnis (1), in das das zu isolierende Zellmaterial im Gewebe- verband und/oder in der Flüssigkeit einbringbar ist,
**dadurch gekennzeichnet, daß** eine stempelartig ausgebildete Trennvorrichtung vorgesehen ist, die einen Betätigungsschaft (3) vorsieht, an dem eine flächig ausgebildete Trennscheibe (2) angebracht ist, deren Umfangsrand fluiddicht mit den Behältnisinnenwänden abschließt und wenigstens eine Durchlaßöffnung aufweist, die von einer Filtermembran (5) überdeckt ist, und die von oben in das Behältnis einbringbar ist, und daß an der Trennscheibe Reibelemente (6) vorgesehen sind, durch die bei Rotation der Trennscheibe Scherkräfte in das Material einbringbar sind.

2. Vorrichtung nach Anspruch 1,
**dadurch gekennzeichnet, daß** eine Verschlußkappe vorgesehen ist, die das Behältnis fluiddicht abschließt.

3. Vorrichtung nach Anspruch 1 oder 2,
**dadurch gekennzeichnet, daß** die Trennscheibe das Zellmaterial samt Gewebeverband und/oder Flüssigkeit druckbeaufschlagt.

4. Vorrichtung nach einem der Ansprüche 1 bis 3,
**dadurch gekennzeichnet, daß** der Betätigungsschaft mittig an der Trennscheibe angebracht ist.

5. Vorrichtung nach einem der Ansprüche 1 bis 4,
**dadurch gekennzeichnet, daß** die Trennvorrichtung bündig zum peripheren Umfangsrand einen Betätigungsschaft von hohlzylindrischer Gestalt aufweist.

6. Vorrichtung nach Anspruch 5,
**dadurch gekennzeichnet, daß** die Trennvorrichtung über den Betätigungsschaft für rotatorische und/oder vertikale Bewegungen antreibbar ist.

7. Vorrichtung nach Anspruch 5 oder 6,
**dadurch gekennzeichnet, daß** das obere Ende des Betätigungsschaftes lösbar fest oder fest an der Verschlußkappe anbringbar ist.

8. Vorrichtung nach einem der Ansprüche 5 bis 7,
**dadurch gekennzeichnet, daß** der Betätigungsschaft einen Flansch zur automatischen oder manuellen Betätigung mittels einer Handhabungsvorrichtung vorsieht.

9. Vorrichtung nach einem der Ansprüche 1 bis 8,
**dadurch gekennzeichnet, daß** die Reibelemente an der dem Boden des Behältnisses zugewandten Seite der Trennscheibe angebracht sind.

10. Vorrichtung nach einem der Ansprüche 2 bis 9,
**dadurch gekennzeichnet, daß** die Verschlußkappe als Schraubdeckel (4) ausgebildet ist.

11. Vorrichtung nach einem der Ansprüche 2 bis 10,
**dadurch gekennzeichnet, daß** die Verschlußkappe ein Septum zum Einstechen mittels Kanülen vorsieht.

12. Vorrichtung nach einem der Ansprüche 1 bis 11,
**dadurch gekennzeichnet, daß** das Behältnis aus Kunststoff gefertigt ist.

13. Vorrichtung nach einem der Ansprüche 1 bis 12,
**dadurch gekennzeichnet, daß** die Filtermembran derart beschaffen ist, daß sie Poren aufweist, die den Durchtritt einzelner Zellen und/oder Zellverbände durch die Membran gewährleisten und das übrige Gewebe zurückhalten.

14. Vorrichtung nach Anspruch 13,
**dadurch gekennzeichnet, daß** die Poren eine Größe von etwa 100 µm aufweisen.

15. Verfahren zur Isolierung von Zellmaterial aus einem Gewebeverband und/oder einer Flüssigkeit unter Verwendung der Vorrichtung nach einem der Ansprüche 1 bis 14,
**gekennzeichnet durch** folgende Verfahrensschritte:
- das zu isolierende Zellmaterial, das in einem Gewebeverband und/oder einer Flüssigkeit integriert ist, wird in das offene Behältnis gegeben,
- die Trennvorrichtung wird derart von oben auf das Zellmaterial samt Gewebeverband und/oder Flüssigkeit in das Behältnis eingebracht, daß die Trennscheibe das Zellmaterial, das in einem Gewebeverband und/oder in einer Flüssigkeit integriert ist, druckbeaufschlagt und mittels rotatorischer Bewegungen das Zellmaterial derart mit Scherkräften beaufschlagt, daß das Zellmaterial zerkleinert wird,
- **durch** Absenken der Trennscheibe in dem Behältnis wird Zellmaterial **durch** die Durchlassöffnungen auf die Oberseite der Trennscheibe gedrückt, übrige Gewebeanteile und/oder Flüssigkeit werden von der Filtermembran im unteren Bereich der Behältnisses zurückgehalten,
- das über der Trennscheibe isolierte Zellmaterial wird mittels Pipettierverfahren und/oder Dekantieren aus dem Behältnis entnommen.

16. Verfahren nach Anspruch 15,
**dadurch gekennzeichnet, daß** vor oder nach Einführen der Trennvorrichtung in das Behältnis Reagenzien zugegeben werden.

## Claims

1. Device for isolating cell material from a tissue formation and/or from a liquid with a receptacle (1) which is open at the top and in which the cell material in the tissue formation and/or in the liquid which is to be isolated can be introduced,
**characterised in that** a separating device is provided which is configured in the fashion of a plunger, which separating device provides an actuation shaft (3) on which there is fitted a two-dimensionally configured separating disc (2), the circumferential edge of which forms a seal in a fluid-impermeable manner with the internal receptacle walls and has at least one admission opening which is covered by a filter membrane (5), and said separating disc can be introduced into the receptacle from above, and **in that** rubbing elements (6) are provided on the separating disc, by means of which shear forces can be introduced into the material during rotation of the separating disc.

2. Device according to claim 1,
**characterised in that** a closing cap is provided which seals the receptacle in a fluid-impermeable manner.

3. Device according to claim 1 or 2,
**characterised in that** the separating disc pressurises the cell material together with the tissue formation and/or liquid.

4. Device according to one of the claims 1 to 3,
**characterised in that** the actuation shaft is fitted centrally on the separating disc.

5. Device according to one of the claims 1 to 4,
**characterised in that** the separating device has an actuation shaft which is in the form of a hollow cylinder and is flush with the peripheral circumferential edge.

6. Device according to claim 5,
**characterised in that** the separating device can be driven via the actuation shaft for rotary and/or vertical movements.

7. Device according to claim 5 or 6,
**characterised in that** the upper end of the actuation shaft can be fitted on the closing cap in a detachably fixed or fixed manner.

8. Device according to one of the claims 5 to 7,
**characterised in that** the actuation shaft provides a flange for automatic or manual actuation by means of a handling device.

9. Device according to one of the claims 1 to 8,
**characterised in that** the rubbing elements are fitted on the side of the separating disc which is orientated towards the base of the receptacle.

10. Device according to one of the claims 2 to 9,
**characterised in that** the closing cap is configured as a screw cover (4).

11. Device according to one of the claims 2 to 10,
**characterised in that** the closing cap provides a septum for piercing by means of cannulae.

12. Device according to one of the claims 1 to 11,
**characterised in that** the receptacle is manufactured from plastic material.

13. Device according to one of the claims 1 to 12,
**characterised in that** the filter membrane is produced such that it has pores which ensure the passage of individual cells and/or cell formations through the membrane and retain the remaining tissue.

14. Device according to claim 13,
**characterised in that** the pores have a size of approximately 100 mm.

15. Method for isolating cell material from a tissue formation and/or from a liquid by using the device according to one of the claims 1 to 14,
**characterised by** the following method steps:
- the cell material to be isolated, which is integrated in a tissue formation and/or in a liquid, is put into the open receptacle,
- the separating device is introduced from above into the receptacle upon the cell material together with the tissue formation and/or liquid in such a manner that the separating disc pressurises the cell material which is integrated in a tissue formation and/or in a liquid, and subjects the cell material to shear forces by means of rotary movements in such a manner that the cell material is reduced in size,
- by means of lowering the separating disc in the receptacle, cell material is forced through the admission openings onto the upper side of the separating disc, remaining tissue components and/or liquid are retained b y the filter membrane in the lower region of the receptacle,
- the cell material isolated above the separating disc is removed from the receptacle by means of pipetting methods and/or decantation.

16. Device according to claim 15,
**characterised in that,** before or after the introduction of the separating device into the receptacle, reagents are added.

## Revendications

1. Dispositif pour isoler une matière cellulaire d'une enveloppe tissulaire et/ou d'un liquide à l'aide d'un récipient (1) dont le dessus est ouvert et dans lequel on place la matière cellulaire à isoler de l'enveloppe tissulaire et/ou du liquide,
**caractérisé en ce qu'**
il comporte un dispositif séparateur en forme de tampon avec une tige d'actionnement (3) munie d'un disque séparateur (2) en forme de surface, dont le bord périphérique est appliqué de manière étanche au fluide avec les parois intérieures du récipient, disque qui comporte au moins un orifice de passage couvert par une membrane de filtre (5) et qui s'introduit par le dessus dans le récipient, et
des éléments de frottement (6) sont prévus sur le disque séparateur pour induire des forces de cisaillement dans la matière par rotation du disque séparateur.

2. Dispositif selon la revendication 1,
**caractérisé par**
un capuchon de fermeture qui ferme le récipient de manière étanche au fluide.

3. Dispositif selon les revendications 1 ou 2,
**caractérisé en ce que**
le disque séparateur applique de la pression à la matière cellulaire y compris au tissu cellulaire et/ou au liquide.

4. Dispositif selon l'une des revendications 1 à 3,
**caractérisé en ce que**
la tige d'actionnement est au milieu du disque séparateur.

5. Dispositif selon l'une des revendications 1 à 4,
**caractérisé en ce que**
le dispositif séparateur comporte à niveau avec le bord périphérique extérieur, une tige d'actionnement en forme de cylindre creux.

6. Dispositif selon la revendication 5,
**caractérisé en ce que**
le dispositif séparateur est entraîné en mouvement de rotation et/ou vertical par la tige d'actionnement.

7. Dispositif selon les revendications 5 ou 6,
**caractérisé en ce que**
l'extrémité supérieure de la tige d'actionnement est reliée de manière solidaire ou amovible au capuchon de fermeture.

8. Dispositif selon l'une des revendications 5 à 7,
**caractérisé en ce que**
la tige d'actionnement comporte une bride pour l'actionnement automatique ou manuel à l'aide d'un dispositif de manipulation.

9. Dispositif selon l'une des revendications 1 à 8,
**caractérisé en ce que**
les éléments de friction sont prévus sur la face du disque séparateur tournée vers le fond du récipient.

10. Dispositif selon l'une des revendications 2 à 9,
**caractérisé en ce que**
le capuchon de fermeture est réalisé comme couvercle vissé (4).

11. Dispositif selon l'une des revendications 2 à 10,
**caractérisé en ce que**
le capuchon de fermeture comporte un septum pour enfoncer à l'aide de canules.

12. Dispositif selon l'une des revendications 1 à 11,
**caractérisé en ce que**
le récipient est en matière plastique.

13. Dispositif selon l'une des revendications 1 à 12,
**caractérisé en ce que**
la membrane de filtre est conçue pour avoir des pores permettant le passage de cellules séparées et/ou de tissus cellulaires à travers la membrane en retenant les autres parties du tissu.

14. Dispositif selon la revendication 13,
**caractérisé en ce que**
les pores ont une dimension d'environ 100 µm.

15. Procédé d'isolation de matières cellulaires d'une enveloppe tissulaire et/ou d'un liquide par application du dispositif selon l'une des revendications 1 à 14,
**caractérisé par** les étapes de procédé suivantes :
- on place dans le récipient ouvert la matière cellulaire à isoler, intégrée dans une enveloppe tissulaire et/ou un liquide,
- on introduit le dispositif séparateur par le dessus dans le récipient sur la matière cellulaire avec l'enveloppe tissulaire et/ou le liquide, on met en pression et avec des mouvements de rotation on induit des forces de cisaillement dans la matière cellulaire pour réduire la matière cellulaire,
- en abaissant le disque séparateur dans le récipient on pousse la matière cellulaire à travers les orifices de passage vers le côté supérieur du disque séparateur, les autres composants tissulaires et/ou de liquide étant retenus par la membrane de filtre dans la zone inférieure du récipient,
- on extrait du récipient la matière cellulaire isolée, au-dessus du disque séparateur en utilisant une pipette et/ou par décantation.

16. Procédé selon la revendication 15,
**caractérisé en ce qu'**
avant ou après introduction du dispositif séparateur dans le récipient on y ajoute des réactifs.
